# FASCICULE DE BREVET EUROPEEN

(11) **EP 0 502 553 B1**
(45) Date de publication et mention de la délivrance du brevet: **30.07.1997**
(21) Numéro de dépôt: 92105747.7
(22) Date de dépôt: 30.04.1986
(51) Int. Cl.: C07K 14/435, C12P 21/08, G01N 33/574, C12N 15/12

(54) **Fragment peptidique de la villine humaine et anticorps la reconnaissant**
Peptidfragment des menschlichen Villins und Antikörper dagegen
Peptide fragment from human villin and antibody thereto

(30) Priorité: 02.05.1985 FR 8506707; 13.11.1985 FR 8516820
(43) Date de publication de la demande: 09.09.1992
(62) Demande divisionnaire de: 86400957.6
(73) Titulaire: INSTITUT PASTEUR, 75724 Paris Cédex 15 (FR); CENTRE NATIONAL DE LA RECHERCHE SCIENTIFIQUE, 75007 Paris (FR); INSTITUT NATIONAL DE LA SANTE ET DE LA RECHERCHE MEDICALE (INSERM), F-75013 Paris (FR)
(72) Inventeur: Louvard, Daniel, F-92330 Sceaux (FR); Dudouet, Brigitte, F-75005 Paris (FR); Robine, Sylvie, F-92170 Vanves (FR); Arpin, Monique, F-75014 Paris (FR); Pringault, Eric, F-75003 Paris (FR); Garcia, Alphonse, F-75015 Paris (FR)
(74) Mandataire: Desaix, Anne

(56) Documents cités:
- JOURNAL OF BIOLOGICAL CHEMISTRY. vol. 206, no. 15 , August 1981 , BALTIMORE US pages 8156 - 8161 GLENNEY, J. ET AL 'demonstration of at least two different actin-binding sites in villin'
- JOURNAL SUBMICROSCOPIC CYTOLOGY vol. 16, no. 1 , January 1984 pages 159 - 160 COUDRIER, E. ET AL 'Expression of two strucural markers of brushborder intestinal mucosa: villin and a membrane glycoprotein (140Kd), in human colon carcinoma cell line HT29'
- CHEMICAL ABSTRACTS, vol. 98, no. 9, February 1983, Columbus, Ohio, US; abstract no. 67511j, DRENCKHAHN, D. ET AL 'evidence for the association of villin with core filiaments and rootlets of intestinal epithelial microvilli' page 244 ;column L ; & CELL TISSUE RES vol. 228, no. 2 , 1983 , GB pages 409 - 14 'evidence for the association of villin with core filiaments and rootlets of intestinal epithelial micrivilli'
- CHEMICAL ABSTRACTS, vol. 100, no. 23, June 1984, Columbus, Ohio, US; abstract no. 189079e, AUSIELLO, D . ET AL 'identification of actin-binding protein and villin in toad bladder epithelial' page 348 ; & AM. J. PHYSIOL vol. 246, no. 1 , June 1984 , US pages F101 - F104 'identification of actin-binding protein and villin in toad bladder epithelia'

## Description

Ceci constitue une division selon l'Article 76 CBE de la demande de brevet européen déposée le 30/04/86 sous le numéro 86400957.6

L'invention est relative à des moyens de diagnostic in vitro de l'existence ou non chez l'homme des proliférations de cellules malignes à partir de cellules tumorales du tube digestif, plus particulièrement du type adénocarcinome. Ces moyens concernent tant des procédés de diagnostic in vitro, que des éléments matériels susceptibles d'être mis en oeuvre dans ces procédés, plus particulièrement des fragments d'ADN utilisables comme sondes ou encore des anticorps, de préférence monoclonaux, dirigés contre la villine humaine.

L'invention étend encore ses effets aux différentes applications de ces anticorps monoclonaux, par exemple à la purification de la villine humaine à partir d'extraits cellulaires la contenant. A ce titre, l'invention concerne aussi la villine humaine purifiée elle-même.

Il a déjà été proposé d'avoir recours à la détection de protéines de structure en tant que marqueurs spécifiques, pour l'identification de certains types de cellules d'eucaryotes supérieurs et pour l'étude du suivi des différentes étapes de leur développement, notamment au cours de leur différenciation. A titre d'exemples de marqueurs connus, on mentionnera des protéines de filaments, par exemple la vimentine, des kératines ou des protéines de neurofilaments, qui ont permis l'identification de certains types de cellules, l'étude de leur développement, à partir du stade de l'embryogénèse jusqu'au stade ultime de la différenciation cellulaire, ainsi que de leur comportement vis-à-vis du milieu environnant (E. LAZARIDES, Nature, 283, 249 (1980) et R. MOLL et al., Cell 31, 11 (1982).

Les kératines sont souvent considérées comme des marqueurs particulièrement efficaces, permettant de distinguer les cellules épithéliales et les cellules non épithéliales. Ceci étant, il a été établi que ce type de distinction ne permettait pas toujours d'établir de façon non ambiguë l'origine des cellules épithéliales en cause.

Pourtant la nécessité d'établir de façon certaine l'origine de certains types de cellules revêt une importance particulièrement importante dans un grand nombre de cas, par exemple lorsqu'il s'agit, à l'occasion de l'examen d'un prélèvement biologique témoignant d'une prolifération de cellules tumorales d'identifier les cellules tumorales primaires, se trouvant à l'origine de cette prolifération, ou encore de détecter l'existence de métastases dont l'origine serait un cancer de la région digestive ou encore de vérifier l'efficacité d'un traitement chimiothérapeutique ou autres contre des métastases cancéreuses. On sait bien que l'efficacité d'un traitement anticancéreux peut être liée à l'identification précise des cellules tumorales qui sont la cause première de la maladie.

Ces observations s'appliquent avec une force particulière à la détection, de préférence précoce, de proliférations de cellules tumorales d'origine digestive (responsables d'une importante proportion des cancers observés en clinique) ou rénales, ou de cellules dérivées des cellules précédentes, plus particulièrement à l'identification de marqueurs caractéristiques de ces cellules.

L'invention s'intéresse donc plus particulièrement à la détection de cellules tumorales qui ont conservé certains des phénotypes essentiels des cellules épithéliales normalement présentes dans les muqueuses de l'appareil digestif, plus particulièrement les muqueuses intestinales, et dont les types différenciés majeurs sont constitués par les cellules absorbantes ou entérocytes.

Plus particulièrement encore l'invention se rapporte à des marqueurs spécifiques susceptibles d'être détectés, qu'ils soient ou non portés par ces cellules tumorales.

En particulier l'invention vise à permettre la détection de ces marqueurs, même lorsqu'ils sont détachés desdites cellules tumorales, notamment à l'occasion de la nécrose de ces cellules, et libérés, par exemple dans la circulation sanguine.

Le marqueur mis en jeu dans le cadre de la présente invention est constitué par tout ou partie de la villine. Il s'agit d'une protéine ayant un poids moléculaire de l'ordre de 95.000 daltons et qui est normalement présente dans les villosités des muqueuses digestives, plus particulièrement intestinales. La villine est capable de se lier à l'actine, en présence d'ions calcium.

Dans un article publié en 1984 (J. Submicrosc. Cytol. 16 (1): 159-160) Coudrier et al ont décrit à partir de l'observation d'une lignée cellulaire de carcinome, la présence de villine dans des cellules différenciées et dans des cellules indifférenciées. La villine a été détectée dans cette lignée cellulaire, au moyen d'anticorps polyclonaux.

D'une espèce à l'autre, la séquence en acides aminés de la villine peut légèrement différer, mais les caractéristiques fonctionnelles sont maintenues. Les techniques de purification connues à ce jour permettent d'obtenir à l'état pratiquement pur la villine de poulet et celle de porc. En revanche ces techniques ne conduisent pas à l'obtention de villine humaine purifiée.

L'une des villines les mieux connues est celle du poulet. Elle est formée d'une séquence de 854 acides aminés. Elle peut être coupée par protéolyse ménagée avec la trypsine et la protéase V-8 en des points déterminés, pour produire des fragments 44 Kd, 51 Kd et 10 Kd dont les positions relatives apparaissent dans la carte, de la villine, d'après Paul MADSUDAIRA, MRC, Laboratory of Molecular Biology, Cambridge, U.K. et John GLENNEY et al, J.B.C., 1981, 256, 8156-8161, qui s'établit comme suit :

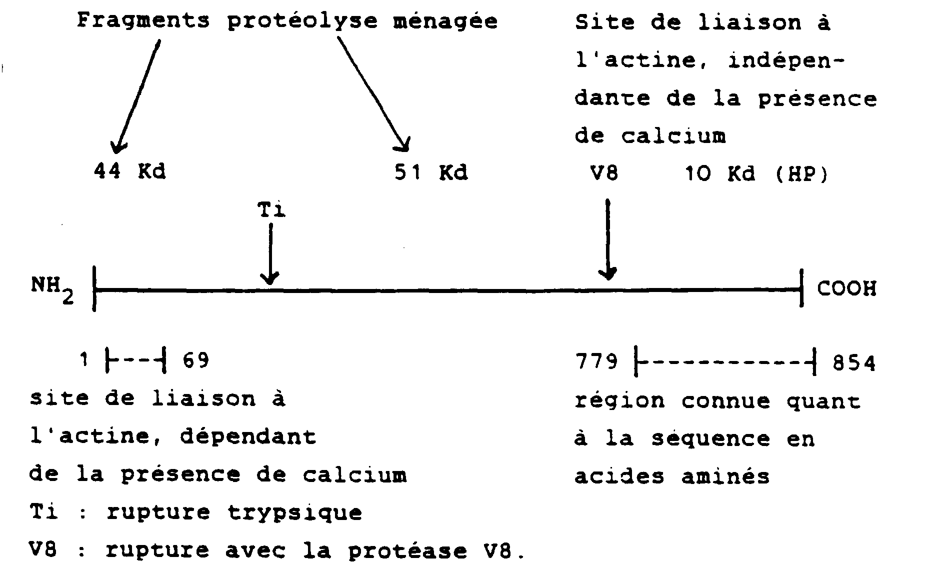
Fragments protéolyse ménagée
Site de liaison à
l'actine, indépen-
dante de la présence
de calcium
44 Kd
51 Kd
V8
10 Kd (HP)
Ti
NH2
COOH
1
69
779
854
site de liaison à
l'actine, dépendant
de la présence de calcium
Ti : rupture trypsique
V8 : rupture avec la protéase V8.
région connue quant
à la séquence en
acides aminés

La séquence en acides aminés de la partie COOH-terminale (779-854) ou "partie de tête" (head piece ou "peptide HP") pour la villine de poulet est la suivante :

1                                       10  11
Val-Phe-Thr-Ala-Thr-Thr-Thr-Leu-Val-Pro-Thr-Lys-Leu-Glu-
                        20
Thr-Phe-Pro-Leu-Asp-Val-Leu-Val-Asn-Thr-Ala-Ala-Glu-Asp-
    30  31                                      40  41
Leu-Pro-Arg-Gly-Val-Asp-Pro-Ser-Arg-Lys-Glu-Asn-His-Leu-
                                50  51
Ser-Asp-Glu-Asp-Phe-Lys-Ala-Val-Phe-Gly-Met-Thr-Arg-Ser-
    60  61                                      70  71
Ala-Asn-Leu-Pro-Leu-Trp-Lys-Gln-Gln-Asn-Leu-Lys-Lys-Glu-

Glu-Lys-Gly-Leu-Phe

D'une manière générale, la villine est présente plus particulièrement dans les bordures en brosses qui sont observées à la surface des entérocytes, notamment lorsque ceux-ci ont atteint leur stade ultime de différentiation, au niveau des parois de la lumière intestinale. Les microvillosités sont des organelles assemblées dans le stade final de la différentiation des entérocytes. La villine est plus particulièrement localisée dans les faisceaux de microfilaments axiaux de ces microvillosités. Elle contribue à la constitution de leur cytosquelette. Par examen de coupes congelées en immunofluorescence, il a pu être constaté que la villine était présente au pôle apical des cellules allongées formant des colonnes affleurant à la paroi interne de l'intestin, et également à proximité des cellules tubulaires proximales du rein. Dans les deux cas il s'agit de régions coïncidant avec la bordure en brosse des cellules concernées. Mais la villine n'a pas été détectée au niveau de divers autres types de villosités de divers autres épithéliums, lorsque celles-ci étaient dépourvues d'une bordure en brosse très organisée (A. BRETSCHER et al, Exp. Cell. res., 135, 213 (1981) ou encore article de H. REGGIO et al, publié dans l'ouvrage intitulé "Membranes in Growth and Development" (Membranes en cours de croissance et de développement) de A. LISS, New York ((1982) pp. 89-105).

Il a en outre été constaté que la villine était présente dans les entérocytes dans un stade précoce de leur développement, bien avant la mise en place de la bordure en brosse.

L'invention résulte d'une double découverte. La villine reste détectable de façon pratiquement systématique au cours de la tumorigénèse des tissus du tube digestif, et plus particulièrement dans les cancers du colon, l'une des formes de cancers parmi les plus répandues et aussi dans certains types de cancers rénaux, alors que la villine n'a jamais été détectée dans des cellules tumorales primaires résultant de la cancérisation initiale de tissus localisés dans d'autres organes (foie, ovaires, poumons, etc.).

En outre, la villine est détectable in vivo à tous les niveaux du développement des cellules tumorales de la région digestive, tant au stade précoce de la tumorigénèse qu'aux stades les plus tardifs, quel que soit l'état de différenciation des cellules en cause.

Le procédé de diagnostic selon l'invention, qui a pour but la détection in vitro de cellules tumorales initialement formées dans la région digestive et/ou dérivées de celles-ci dans un prélèvement biologique est caractérisé par la mise en contact de ce prélèvement biologique avec des réactifs présentant une affinité spécifique pour la villine ou reconnaissant le gène codant pour cette protéine.

Le procédé de diagnostic selon l'invention est applicable à tout échantillon biologique susceptible de contenir des cellules tumorales d'origine digestive ou des cellules tumorales éventuellement formées dans d'autres régions de l'organisme, sous l'influence de cellules primaires d'origine digestive ou rénale, ou encore à tout échantillon biologique susceptible de contenir des produits de dégradation de ces diverses cellules tumorales, y compris le marqueur villine, à l'occasion de la nécrose desdites cellules. Cet échantillon biologique peut consister en toutes formes de prélèvements : fragments de tissus ou de tumeurs solides, par exemple obtenus par biopsie, ou encore prélèvement de liquides biologiques, plus particulièrement de sang total ou de serum sanguin, susceptible de véhiculer des cellules malignes ou des fragments originaires de celles-ci.

Selon un mode de réalisation de l'invention, le réactif mis en oeuvre dans le procédé de diagnostic de l'invention est constitué par un ADN dont la séquence comporte une région codant pour une séquence d'acides aminés de la villine humaine, plus spécialement la partie COOH-terminale.

Selon un autre mode de réalisation de l'invention, les techniques mises en oeuvre dans le procédé de diagnostic font appel à des techniques immunologiques, le réactif présentant une affinité spécifique pour la villine étant alors constitué par des anticorps susceptibles de reconnaître la villine.

Ces anticorps sont eux-mêmes marqués de toute façon en soi connue, ou sont reconnaissables à leur tour, lorsqu'ils sont à l'état fixé sur la villine ou les tissus la portant, par des immunoglobulines marquées ou autres protéines marquées (par exemple la protéine A de Staphylococcus aureus).

Des techniques particulièrement appropriées sont celles qui ont été mises en oeuvre dans les essais qui seront décrits plus loin, et qui ont conduit aux constatations exposées ci-dessus.

L'invention a également pour but de fournir des moyens nouveaux spécifiques, pour la détection de la villine au niveau des microvillosités des entérocytes ou cellules analogues (étant entendu que l'utilisation de ces moyens nouveaux n'est pas nécessairement limitée à la mise en oeuvre du procédé de diagnostic conforme à l'invention).

Selon un aspect de l'invention, ces moyens sont constitués par l'ADN correspondant à l'ARNm complet de la villine humaine ou par un fragment d'ADN dont la séquence nucléotidique comporte une région codant pour une séquence d'acides aminés de la villine humaine, capable de s'hybrider spécifiquement avec une séquence nucléotidique codant pour la villine.

L'invention vise plus spécialement l'utilisation dans le procédé ci-dessus d'un ADN correspondant à l'ARNm complet de la villine humaine ou d'un fragment d'ADNc caractérisé par le fait qu'il renferme au moins une partie de la séquence nucléotidique codant pour les acides aminés de l'extrémité COOH de la villine humaine. Cette séquence nucléotidique et la séquence correspondante des acides aminés codés sont indiquées ci-après :

LYS TRP SER ASN THR LYS SER TYR GLU ASP LEU LYS ALA GLU
AAG TGG AGT AAC ACC AAA TCC TAT GAG GAC CTG AAG GCG GAG

SER GLY ASN SER ARG ASP TRP SER GLN ILE THR ALA GLU VAL
TCT GGC AAC TCT AGG GAC TGG AGC CAG ATC ACT GCT GAG GTC

THR SER PRO LYS VAL ASP VAL PHE ASN ALA ASN SER ASN LEU
ACA AGC CCC AAA GTG GAC GTG TTC AAT GCT AAC AGC AAC CTC

SER SER GLY PRO LEU PRO ILE PHE PRO LEU GLU GLN LEU VAL
AGT TCT GGG CCT CTG CCC ATC TTC CCC CTG GAG CAG CTA GTG

ASN LYS PRO VAL GLU GLU LEU PRO GLU GLY VAL ASP PRO SER
AAC AAG CCT GTA GAG GAG CTC CCC GAG GGT GTG GAC CCC AGC

ARG LYS GLU GLU HIS LEU SER ILE GLU ASP PHE THR GLN ALA
AGG AAG GAG GAA CAC CTG TCC ATT GAA GAT TTC ACT CAG GCC

PHE GLY MET THR PRO ALA ALA PHE SER ALA LEU PRO ARG TRP
TTT GGG ATG ACT CCA GCT GCC TTC TCT GCT CTG CCT CGA TGG

LYS GLN GLN ASN LEU LYS LYS GLU LYS GLY LEU PHE *
AAG CAA CAA AAC CTC AAG AAA GAA AAA GGA CTA TTT TGA GAAG
AGTAGCTGTGGTTGTAAAGCAGTACCCTACCCTGATTGTAGGGTCTCATTTTCTCA
CCGATATTAGTCCTACACCAATTGAAGTGAAATTTTGCAGATGTGCCTATGAGCAC
AAACTTCTGTGGCAAATGCCAGTTTTGTTTAATAAATGTACCTATTCCTTCAGAAA
GATGATACCCCAAAAAAAAAAAA

La séquence nucléotidique mise en oeuvre est utilisée comme sonde pour la détection des ARNm ou des ADN codant pour la villine humaine.

Une sonde appropriée pour ce type de détection est avantageusement marquée par un élément radioactif ou tout autre groupe permettant sa reconnaissance à l'état hybridé avec la préparation renfermant l'ARNm ou l'ADN à étudier.

Selon les techniques classiques, ces sondes sont mises en contact avec un prélèvement biologique renfermant les cellules à étudier, ou directement avec leurs acides nucléiques, dans des conditions autorisant l'hybridation éventuelle de la séquence nucléotidique de la sonde avec une séquence complémentaire éventuellement contenue dans l'échantillon testé.

La reproductibilité de la détection a été testée en utilisant une sonde contenant un insérat de 200 paires de bases.

Ces sondes nucléotidiques, qui constituent des produits nouveaux entrant dans le cadre de l'invention, sont utilisées également, selon un autre aspect, pour l'étude de l'expression des ARNm de la villine dans chacun des états de différenciation des entérocytes et/ou des cellules exprimant la villine, et dans les différents stades de maturation des cellules d'origine rénale ou intestinale.

Les sondes obtenues permettent ainsi de déterminer le nombre, l'organisation et la structure du ou des gènes de la villine tels qu'isolés à partir d'une banque génomique humaine.

Conformément à l'invention, on isole la séquence nucléotidique codant pour l'extrémité de la villine humaine en procédant selon les étapes suivantes :
- on prépare selon les techniques connues les ARNm (ARN messagers) totaux à partir d'une lignée cellulaire exprimant la villine ;
- on construit une banque d'ADNc (ADN complémentaires) ;
- on insère les ADNc dans un vecteur susceptible d'exprimer la protéine codée par l'insérat ;
- on transforme à l'aide des vecteurs recombinants obtenus une souche bactérienne, puis on établit des conditions permettant l'expression de la protéine recherchée dans la bactérie ;
- on sélectionne les clones recombinants contenant le clone spécifique de la villine à l'aide d'anticorps reconnaissant la villine.

L'étape de construction de la banque d'ADNc est réalisée à l'aide d'un vecteur capable d'exprimer la protéine codée par l'ADNc inséré.

Des vecteurs de clonage particulièrement avantageux, en raison notamment de leur rendement d'expression élevé, sont constitués par des plasmides de type pEX. De tels plasmides sont décrits par Stanley et Luzio dans EMBO Journal, 1984, 3, 1429-1434. Ils dérivent de plasmides contenant le gène de fusion cro-lacZ dont l'expression est sous le contrôle du promoteur P_{R} du bactériophage lambda. Cette expression est inductible par la température.

Un polynucléotide contenant plusieurs sites uniques de restriction est inséré dans chacune des phases de lecture, ainsi que les signaux de terminaison de la transcription et de la traduction. Les ADNc insérés au niveau des sites uniques de restriction sont exprimés avec une grande efficacité sous forme de protéine hybride cro-βgal-peptide correspondante. Cette protéine hybride est peu soluble, ce qui réduit d'une manière avantageuse les risques de protéolyse dans la bactérie et facilite sa détection immunologique par des anticorps spécifiques.

Pour réaliser l'insertion de l'ADNc dans la bonne orientation par rapport au gène cro-lacZ, afin d'obtenir l'expression dans la bactérie du polypeptide correspondant, on utilise un protocole d'addition séquentielle de linkers différents aux deux extrémités de l'ADNc selon la technique de Helfmann et al. dans P.N.A.S. USA, 1983, 80, 31-35. Les linkers utilisés correspondent aux sites de coupure de BamHI et SalI.

Ces plasmides recombinants sont utilisés pour transformer, selon les techniques classiques, la souche bactérienne mise en oeuvre pour l'expression de la protéine par la séquence recherchée.

Les souches bactériennes de E. coli sont particulièrement préférées, plus spécialement la souche E. coli pop 2135 qui contient l'allèle cIts857 du répresseur cro.

On obtient ainsi dans la souche bactérienne une banque d'ADNc correspondant à l'ARNm des cellules exprimant un haut niveau de villine, contenant environ 30 000 clones recombinants.

L'expression de la protéine hybride est inductible par la température. On opère donc à une température où le répresseur du gène cro n'est plus actif. A cet effet, il est satisfaisant d'incuber les souches environ 2 heures à une température supérieure à l'ambiante, en particulier de l'ordre de 40-42°C.

Les clones récupérés après lyse des bactéries et renaturation des protéines selon les techniques classiques sont sélectionnés par criblage immunologique.

D'une manière préférée, on effectue d'abord un criblage de la banque d'ADNc par au moins un type d'anticorps polyclonal anti-villine. Les clones réagissant spécifiquement avec le ou les anticorps polyclonaux sont recriblés à l'aide d'au moins un type d'anticorps monoclonal dirigé contre des épitopes COOH-terminaux.

Un criblage satisfaisant est réalisé à l'aide d'un anticorps anti-villine polyclonal tel qu'un anticorps polyclonal dirigé contre la villine de porc intacte puis un ou plusieurs anticorps monoclonaux reconnaissant des épitopes situés dans la région COOH-terminale de la molécule.

Le criblage à l'aide de l'anticorps polyclonal est avantageusement suivi d'un criblage secondaire à l'aide d'un autre anticorps polyclonal, en particulier un anticorps polyclonal dirigé contre le fragment COOH-terminal de la villine de poulet.

L'invention vise plus particulièrement un clone porteur d'un ADNc correspondant à la villine humaine caractérisé en ce qu'il réagit spécifiquement avec l'anticorps polyclonal dirigé contre la villine intacte, avec l'anticorps polyclonal dirigé contre le peptide COOH-terminal de la villine de poulet et avec les deux anticorps monoclonaux dirigés contre des épitopes COOH-terminaux.

L'invention vise également un clone porteur d'un ADNc correspondant à la villine comprenant un insérat avec une séquence polyA et un site de poly-adénylation.

Selon un autre aspect de l'invention, les moyens mis en oeuvre pour la détection de la villine sont constitués par des anticorps monoclonaux.

Cet aspect de l'invention s'appuie sur le fait que les villines, originaires de différentes espèces, possèdent des épitopes communs, accessibles à des anticorps monoclonaux reconnaissant de façon spécifique non seulement la villine particulière ayant servi à l'immunisation de l'animal, dont les cellules spléniques avaient été fusionnées avec les cellules de myélome appropriées à la production des hybridomes secréteurs desdits anticorps monoclonaux, mais reconnaissant de façon aussi spécifique des villines originaires d'autres espèces, y compris l'espèce humaine.

Un anticorps monoclonal préféré selon l'invention est donc caractérisé par les propriétés suivantes :
- Il reconnaît la villine de porc purifiée (Western Blotting).
- Il reconnaît la villine de poulet (Western Blotting).
- Il reconnaît la villine d'extrait cellulaire de la lignée dérivée d'un adénocarcinome de colon humain : HT29 (Western Blotting).
- Il reconnaît la villine de rat en immunocytochimie (coupes congelées (cryostat) de muqueuse intestinale de rat fixées au formaldéhyde).

Un anticorps monoclonal préféré parmi ceux qui répondent à la définition précédente reconnaît également un épitope contenu dans le "peptide HP" de la villine de poulet, la reconnaissance étant possible tant à l'égard du peptide HP préalablement isolé qu'à l'égard du peptide 51Kd contenant encore le peptide HP ; cet anticorps ne reconnaît ni le peptide 44Kd ni le peptide 44Kd de la villine de poulet.

L'anticorps préféré de l'invention reconnaît d'ailleurs la protéine HP, aussi bien à l'état dénaturé, notamment après traitement par le dodécylsulfate de sodium (SDS) que sur les cellules épithéliales porteuses de la villine. Ce résultat démontre que l'épitope porté par le peptide HP n'est pas masqué par d'autres protéines dans le milieu cellulaire.

L'invention concerne naturellement également les hybridomes secréteurs de ces anticorps monoclonaux. Une technique avantageuse pour produire ces hybridomes, notamment par fusion de cellules spléniques de souris immunisées contre la villine purifiée de porc, d'une part, et de cellules d'un myélome approprié, sera décrit plus loin. L'hybridome préféré ainsi obtenu (souche BD-D 2C3) a été déposé à la Collection Nationale des Cultures de Microorganismes (CNCM) de l'Institut Pasteur de Paris, le 29 Avril 1985, sous le n° I-440.

Selon un aspect avantageux de l'invention, l'agent immunogène utilisé pour former des anticorps peut être exprimé en grandes quantités dans la bactérie. Cet agent immunogène correspond au peptide codé par la séquence nucléotidique définie ci-dessus correspondant à l'ADN de la villine humaine ou à un fragment.

Selon les techniques classiques, on injecte le peptide à des animaux, on récupère les antisérums, puis, le cas échéant, les anticorps à partir des antisérums, par exemple par chromatographie d'affinité. La production d'anticorps selon cette variante revêt un intérêt d'autant plus grand que la région HP codée par le fragment d'ADNc définie plus haut est à la fois la région la plus immunogène et la plus spécifique de la villine.

On remarquera que la protéine codée par la séquence nucléotidique de l'ADN complémentaire de la villine ou d'un fragment de cet ADN constitue également une source d'antigènes permettant de réaliser des radioimmunoassays compétitifs (soit par la méthode ELISA directe, soit par la méthode ELISA par compétition).

Il va cependant de soi que tout autre agent immunogène contenant la même séquence immunogène peut être utilisée ; par exemple, le peptide HP lui-même, le cas échéant greffé au préalable sur une molécule porteuse, telle que la sérumalbumine bovine, en vue d'accroître ses propriétés immunogènes.

Il apparaîtra clairement à l'homme du métier qu'ayant en mains l'anticorps monoclonal préféré selon l'invention, il est à même de définir la région épitopique caractéristique du peptide HP. Pour l'identifier de façon plus précise, il pourrait notamment avoir recours à un procédé comprenant les étapes suivantes :
- synthèse d'une séquence polynucléotidique codant pour le peptide HP (ou pour la partie de peptide HP dont il est raisonnable de présumer qu'elle contient l'épitope recherché),
- linéarisation dudit plasmide défini ci-dessus contenant la séquence nucléotidique codant pour l'extrémité COOH-terminale de la villine, et ce au niveau d'un site de restriction extérieur à ladite séquence,
- émondage de façon contrôlée du plasmide linéarisé avec une enzyme exonucléolytique, telle que l'enzyme Bal 31,
- recircularisation du plasmide au moyen d'une ADN-ligase,
- transformation d'un microorganisme approprié, lui-même transformable par le plasmide correspondant et capable d'exprimer l'insérat contenu dans celui-ci,
- remise en contact des produits d'expression de ce microorganisme avec l'anticorps considéré,
le cycle d'opérations qui vient d'être défini étant répété jusqu'à la disparition de la détection dudit peptide immunogène parmi les produits d'expression dudit microorganisme transformé par le dernier plasmide recircularisé.

Il est possible, au terme de chacun des cycles du procédé sus-défini, notamment séquençage des parties d'extrémité du plasmide avant et après la dernière opération d'émondage, ayant conduit à la perte de capacité de reconnaissance du plasmide recircularisé intermédiaire, de situer l'épitope au niveau du peptide codé par la séquence nucléotidique éliminée au cours de cette dernière opération d'émondage. C'est dire que le fait pour l'homme du métier de disposer de l'anticorps monoclonal sus-indiqué est équivalent à la mise à sa disposition de la séquence peptidique définie chimiquement et contenant cet épitope.

L'invention concerne également la villine humaine pratiquement pure biologiquement, réagissant avec l'anticorps monoclonal préféré défini ci-dessus et ne donnant essentiellement qu'une seule bande dans des essais de migration en électrophorèse sur gel de polyacrylamide, notamment SDS-PAGE.

En effet, la villine humaine peut être extraite d'un lysat d'entérocytes humains, notamment obtenu par traitement de ces derniers avec une solution aqueuse contenant un détergent approprié, et purifiée à l'aide du susdit anticorps monoclonal. Un procédé de purification de ce type met en jeu l'anticorps monoclonal avantageusement immobilisé sur un support solide, de préférence adapté à des opérations de chromatographie d'affinité. Par exemple, l'anticorps monoclonal est fixé sur un réseau d'agarose à réticulation tri-dimensionnelle, commercialisé sous la marque SEPHAROSE par la société suédoise PHARMACIA A.G., par exemple par la méthode au bromure de cyanogène.

L'invention concerne donc plus particulièrement un procédé de séparation de la villine humaine caractérisé par les opérations consistant à faire passer une solution la contenant au contact d'une colonne d'affinité portant le susdit anticorps monoclonal, pour fixer sélectivement la villine humaine, puis à récupérer celle-ci par dissociation du complexe antigène-anticorps soit par tampon acide à pH 2-4 à base de glycine, soit par un tampon basique à base de méthylamine, pH 11, puis à dialyser contre un tampon d'acétate d'ammonium.

Il va de soi que l'invention concerne également des polypeptides de poids moléculaires plus faibles consistant en des fragments de villine humaine. Il apparaîtra clairement au spécialiste que les fragments pourront être obtenus par découpage de la villine humaine par des enzymes de coupure de polypeptides en des sites spécifiques. A titre d'exemple de telles protéines, on mentionnera en premier lieu la trypsine ou la protéase de Staphylococcus aureus V8, l'alphachymotrypsine, la "protéase de glande sous-maxillaires de souris" (mouse submaxillary gland protease) commercialisée par la Société BOEHRINGER, la collagénase de Vibrio alginolyticus chemovar iophagus, qui reconnaît spécifiquement lesdits peptides Gly-Pro et Gly-Ala, etc.

Il va sans dire que font alors également partie de l'invention les hybridomes et anticorps monoclonaux spécifiques d'espèce ou non qui peuvent être fabriqués à partir de la villine humaine ou de ses fragments,

D'autres caractéristiques et avantages de l'invention apparaîtront encore dans la description qui suit des conditions particulières, indiquées à titre d'exemple, de détection de villine humaine, de production et de mise en oeuvre dans des essais d'hybridomes et d'anticorps monoclonaux.

On rapporte également un procédé d'obtention d'un clone porteur d'une portion d'ADNc de la villine humaine.

### EXEMPLE 1 : DETECTION DE VILLINE HUMAINE.

L'expression de la villine peut être étudiée à différents stades de la prolifération de cellules tumorales ou non originaires des régions digestives ou rénales d'une part, et d'autres régions de l'organisme d'autre part. Partant des tissus concernés, ceux-ci ont été congelés, fixés et préparés dans des conditions à permettre la réalisation de coupes (cryocoupes) selon la technique décrite par BROWN et FARQUHAR, Cell. 36, 295 (1984).

Des coupes ayant des épaisseurs de 5 µm ont été obtenues à -25°C et déposées sur des lames de verre recouvertes de polylysine. Après trempage dans une solution d'un tampon constitué d'une solution saline de phosphate et contenant 0,2 % de gélatine (PBS-gélatine), les coupes ont été traitées pour détecter la villine à l'aide d'une solution contenant 50 µl d'antisérum de lapin antivilline dilué au 1/800° dans la PBS-gélatine, puis incubées pendant 25 minutes à la température ambiante dans une chambre humide.

Les coupes ont ensuite été lavées trois fois pendant 10 minutes dans la PBS-gélatine.

Des anti-IgG de lapin marqués par la rhodamine (produit par la Société néerlandaise Nordic Immunological Laboratories) ont ensuite été ajoutés et les coupes incubées en présence de ces anti-IgG à la température ambiante pendant 25 minutes. Les coupes ont ensuite été lavées de façon complète dans la PBS-gélatine, puis montées pour examen sous microscope à fluorescence (Photomicroscope ZEISS) équipé de lentille à immersion dans une huile Plan Néofluar et d'un jeu de filtres appropriés.

Les tissus témoins ou les tissus tumoraux d'autres origines ont été traités de la même manière.

Les observations suivantes ont pu être faites:

Lorsque les tissus provenaient d'adénocarcinomes du colon, 11 observations sur 12 se sont révélées positives pour ce qui est de l'expression de la villine. Des tumeurs humaines d'autres origines telles que des lymphomes, notamment lymphomes de nodules mésentériques et des types variés de carcinomes d'autres origines résultant des métastases ou non au niveau du pancréas, de l'oesophage, n'ont pas exprimé la protéine.

D'une façon générale, les observations ont montré une corrélation étroite entre l'origine intestinale primaire de tumeurs traitées et l'expression de la villine. Les nombreux essais réalisés sur des tumeurs manifestement d'origine distincte se sont pratiquement dans tous les cas révélés négatifs au niveau de l'expression de la protéine.

Il est à remarquer que dans les tissus d'origine intestinale, la villine a toujours été observée, quel que soit le degré de prolifération desdites cellules.

Les essais qui précèdent ont été réalisés avec des anticorps polyclonaux. Des résultats beaucoup plus nets peuvent être obtenus avec l'anticorps monoclonal préféré qui a fait l'objet d'un dépôt à la CNCM.

Les conditions dans lesquelles les hybridomes secréteurs de ces anticorps monoclonaux ont été isolés seront décrites ci-après à titre d'exemples. Il sera également indiqué un mode de mise en oeuvre préféré des conditions dans lesquelles le diagnostic de la présence ou non de villine peut être détecté dans des essais immunologiques du type ELISA.

### EXEMPLE 2 : PREPARATION D'ANTICORPS MONOCLONAUX DIRIGES CONTRE LA VILLINE.

### A) PROGRAMME D'IMMUNISATION

- Souris Balb/C o 6 à 8 semaines.
- Antigène : villine de porc purifiée (10 mg/ml) (bande homogène après électrophorèse de la protéine sur gel de polyacrylamide (10 %) en présence de SDS de poids moléculaire 95Kd) :
   . 10 mM Imidazole
   . 75 mM HCl
   . 1 mM EGTA
   . 0,1 M DTT
   . 50 % Glycérol.
- Programme :
   . Jour 0 : Injection en IP de 50 µg de villine pure dans une émulsion 50/50 (PBS-Adjuvant de Freund Complet).
   . Jour 14 : Rappel en IP 50 µg de villine (émulsion PBS-Adjuvant de Freund Incomplet).
   . Jour 21 : Rappel en IP 50 µg de villine (émulsion PBS-Adjuvant de Freund Incomplet).
   . Jour 28 : Injection en IM de 20 µg de villine pure en solution dans du PBS.
   . Jour 29 : Injection IV de 10 µg de villine pure en solution dans du PBS.
   . Jour 32 : Fusion.

### B) FUSION

I) Cellules parentales
   a) Cellules de myélome :
      . Lignée Sp2/O-Ag14 (8 Azaguanine résistant).
      . Culture stérile en milieu DMEM 10 % FCS.
   b) Cellules spléniques :
      Origine : rate de souris o Balb/C hyperimmunisée contre la villine de porc.
II) Procédé de fusion cellulaire selon Kùhler G. et Milstein C. (Continuous culture of fused-cells secreting antibody of predefined specificity, Nature 1975, 256, 495).
   - Fusion en milieu DMEM sans sérum dans des conditions stériles, en présence d'une solution de polyéthylèneglycol (PEG 1000-Merck 9729) à 50 % dans un milieu DMEM sans sérum. Après dénombrement des deux types de cellules parentales, la suspension de cellules de myélome en culture est mélangée à la suspension de cellules spléniques dans la proportion de 1 pour 5.
   - Contact 2'30".
   - Arrêt de l'action du PEG par dilution en milieu DMEM complet.
   - Dilution finale de la suspension cellulaire (2x10⁵ cellules/ml) dans du milieu sélectif DMEM-HAT.
   - Distribution dans puits boîte COSTAR-24 trous 1 ml/puits (COSTAR Tissue Culture Cluster 24, Cat. N° 3524, COSTAR 205 Groadway, Cambridge, Ma. USA).
III) Sélection des clones
   Les clones sont identifiés pour leur capacité à sécréter des anticorps dirigés contre la villine purifiée de porc.

### C) METHODE DE SELECTION

I) Test ELISA : Ce test permet la mise en évidence d'anticorps monoclonaux anti-villine dans les surnageants de culture après fusion.
   - Fixation de l'antigène sur support (plaque ELISA) Concentration : 5 µg/ml dans un tampon phosphate de potassium 50 mM pH 8, 1 nuit à 4°C.
   - Saturation avec PBS-Tween 20-BSA.
   - Incubation I avec surnageant d'hybridome non dilué, 3 heures à 4°C.
   - Incubation Il avec des anti-IgG de souris marquées à la bêta-galactosidase, 2 heures à 4°C.
      Tous les lavages entre chaque étape sont effectués avec du PBS-Tween 20 0,1 %.
   - Substrat O-nitrophényl-bêta-D-galactopyranoside (Sigma N11-27) dans du tampon phosphate 0,1 M pH 7,0. 10⁻³ M MgSO₄. 2x10⁻³ M MnSO₄. 2x10⁻³ M magnésium Tritriplex déshydraté (Merck 8409).
      Lecture : DO 414 nm.

   Les clones sélectionnés sont ceux pour lesquels les surnageants d'hybridome donnent une DO supérieure à 10 fois la DO du bruit de fond.
II) Western Blotting selon Burnette. Anal. Biochem. 1981, 112, 195-203.

Ce test permet de sélectionner les clones sécrétant des anticorps monoclonaux anti-villine de porc, reconnaissant également la villine humaine et la villine de poulet, parmi les clones positifs pour le test ELISA.

### Différentes étapes du test :

a) Electrophorèse sur gel de polyacrylamide d'un extrait cellulaire de la muqueuse intestinale de poulet et d'un extrait cellulaire de la lignée HT29 (adénocarcinome de colon humain) exprimant la villine.
b) Electrotransfert des protéines séparées sur gel de polyacrylamide sur papier de nitrocellulose.
c) Incubation du papier de nitrocellulose sur lequel les protéines cellulaires ont été transférées avec les surnageants d'hybridomes préalablement sélectionnés en ELISA 1 nuit à 4°C.
d) Incubation 1 heure à température ambiante avec des anti-IgG de souris marquées à la peroxydase.
e) substrat :
   - 10 mg tétrahydrochlorure de diaminobenzidine.
   - 20 ml 0,1 M Tris HCl pH 7,6.
   - 0,2 ml H₂O₂ à 1 %.

   Tous les lavages entre chaque étape sont effectués avec du sérum de veau nouveau-né-PBS-Triton X100.
f) réaction positive : apparition rapide d'une bande marron (poids moléculaire 95Kd) si la réaction antigène-anticorps monoclonal a eu lieu.

### D) CLONAGE DES HYBRIDES SELECTIONNES

- Méthode des dilutions limitées :
   Dilution en milieu sélectif des clones positifs de façon à ne distribuer qu'une cellule par puits (plaques 96 trous) sur des macrophages (origine souris Balb/C, 4 semaines) attachées au fond des puits.
   Addition de milieu sélectif conditionné.
- Sélection des clones positifs selon les méthodes précédemment énoncées.
- Reclonage si nécessaire.

### E) PREPARATION D'ASCITES

- Les clones sélectionnés sont injectés à des souris (qui ont subi une injection de "PRISTANE" (Aldrich, 2,6,10,14-tétraméthylpentadécane) 4 à 5 jours avant).
- 1 à 2x10⁶ cellules injectées/souris.
- Récupération au bout de 10 à 15 jours du liquide d'ascite contenant les cellules du clone qui ont proliféré. Le liquide d'ascite a un taux d'anticorps monoclonal anti-villine supérieur à 1 mg/ml.

= Congélation des clones sélectionnés en milieu DMEM 10% FCS-5% DMSO.
Conservation dans azote liquide à -176°C.

### PROCEDE ELISA DIRECT POUR DOSER LA VILLINE.

= Adsorption des IgG purifiées à partir de l'ascite BD-D_{2C3} en concentration constante sur des plaques ELISA.
   . L'anticorps a été purifié par chromatographie d'échange d'ions (DEAE-Tris acryl) et sur colonne d'hydroxyapatite.
   . Concentration à établir.
   . Incubation 2 heures à 37°C, puis 1 nuit à 4°C.
   . Lavage dans PBS/Tween 20 0,1 %.
= Saturation des plaques
   Incubation 30 minutes en présence de PBS/Tween 20 0,1%/BSA 0,4%.
= Addition de l'antigène
   . Gamme de concentration décroissante de la villine purifiée de 5 µg/ml à 1 ou 0,1 ng/ml en présence de BSA 0,4 %.
   . Incubation 3 heures à 4°C.
   . Lavages dans PBS/Tween 20 0,1 %.
= Addition des IgG purifiées à partir d'un sérum polyclonal de lapin dirigé contre la villine et couplées à la bêta-galactosidase.
   . Incubation 2 heures à 4°C.
   . Lavages dans PBS/Tween 20 0,1 %.
= Détection de la bêta-galactosidase
   . Addition de 20 mg de p-nitrophényl-bêta-D-galactopyrannoside à partir d'une solution à 4 mg/ml, dans 20 ml de tampon phosphate 0,1 M pH 7, MgSO₄ 10⁻³ M, MnSO₄ 2x10⁻⁴ M, EDTA (Merck 8409) 2x10⁻³ M.
   . Incubation x heures à 37°C.
   . Lecture densité optique à 414 nm.

### EXEMPLE 3 : OBSERVATIONS EN CLINIOUE HUMAINE.

En utilisant un test ELISA décrit ci-après dont la sensibilité permet de détecter des taux très faibles de villine dans un extrait cellulaire (0,5 ng de villine par mg de protéines totales), on a effectué des mesures pour déceler des quantités dosables de villine dans le sérum. En effet, la villine étant une protéine intracellulaire, exprimée par les cellules normales et tumorales du tube digestif, les inventeurs ont vérifié que dans certaines conditions physiopathologiques, la nécrose des cellules contenant de la villine peut conduire à sa libération dans la circulation sanguine.

L'étude dans une population de donneurs de sang (n=190) montre que la villine n'est pas détectable dans la très grande majorité des sérums (n=168) ; cependant un petit nombre d'individus (n=15) présente un taux de villine décelable (5 à 10 ng/ml). Cette valeur est retenue comme le niveau de villine n'ayant pas de signification pathologique. Enfin, quelques individus (n=7) ont un niveau de villine supérieur à la valeur de base (50 à 100 ng/ml). Ces individus dit "normaux" (3,6%) n'ont pas été soumis à un examen clinique complémentaire de leurs voies digestives (fibroscopie, colonoscopie).

Les premiers résultats obtenus dans différentes pathologies gastro-intestinales sont résumés sur le tableau ci-après.

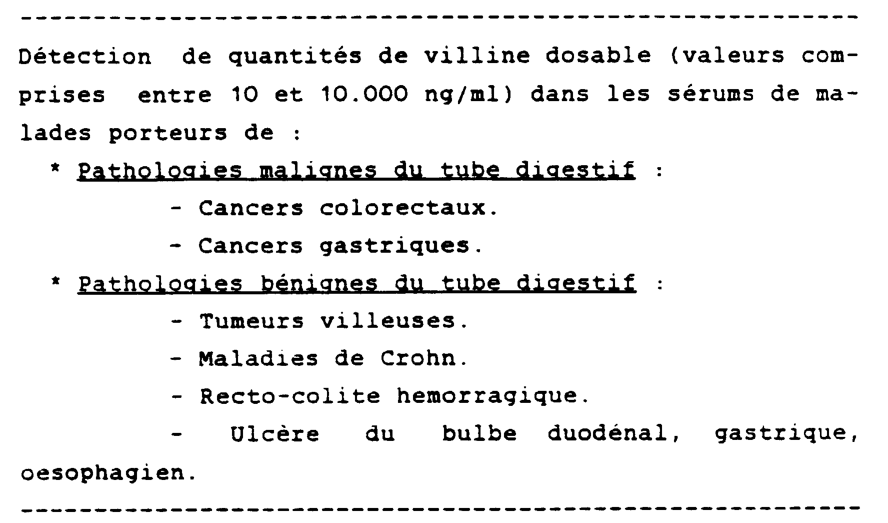

Il est important de noter que par contre, les polyadénomes ("polypes") digestifs ne conduisent pas à la libération de cette protéine dans le sang.

Certains points complémentaires ont pu être déduits de cette étude :
1/ Il ne semble pas exister de corrélation entre la quantité villine circulante et la taille ou le stade évolutif de la tumeur.
2/ Lors du suivi post-opératoire, le dosage de cette protéine montre une chute de son taux sérique après exerèse complète. Par contre, en cas de persistance de tissu tumoral (exerèse incomplète, récidives, metastases), il est observé soit un niveau de villine maintenu, soit une augmentation de celui-ci.

Cette étude indique que la villine n'est pas habituellement détectable dans les sérums des sujets normaux, tandis que l'on peut la doser dans les sérums de malades atteints de pathologies malignes (cancers coliques ou du rectum) ou ayant des ulcérations bénignes (type maladies de Crohn, RCH, ulcère) du tube digestif faisant intervenir des processus inflammatoires et nécrotiques des muqueuses digestives.

Le dosage sanguin de la villine s'avère également d'un grand intérêt dans la surveillance des maladies bénignes (ulcérations et maladies inflammatoires des voies digestives). D'autre part, pour la recherche de villine circulante, il est possible d'utiliser, en association avec les anticorps de l'invention, d'autres marqueurs tumoraux comme l'ACE (antigène carcinogénique embryonnaire) qui est actuellement utilisé pour la surveillance évolutive des cancers mais dont les inconvénients principaux sont : 1/ d'être peu spécifique de l'organe atteint ; 2/ de s'observer dans des pathologies bénignes variées.

### EXEMPLE -4: PREPARATION D'ADNc CORRESPONDANT A LA VILLINE HUMAINE.

### Préparation des ARNm.

On prépare les ARNm à partir de la lignée cellulaire HT29 dérivée d'un adénocarcinome colique humain. Les ARN sont extraits par la méthode au chlorure de guanidium décrite par Ullrich et al. dans Science, 1977, 196, 1313-1319. La purification des ARNm polyA⁺ est effectuée par chromatographie sur colonne d'oligo dT-cellulose selon la méthode décrite par Aviv et Leder dans Proc. Natl. Acad. Sci. USA, 1972, 69, 1408-1412.

### Préparation des ADNc.

Le premier et second brin des ADNc sont préparés selon la méthode de Fiddes et Goodman décrite dans Nature, 1979, 281, 351-356. L'addition séquentielle des linkers SalI à l'extrémité 3' et Bam H1 à l'extrémité 5' est effectuée par la méthode d'Helfmann et al. rapportée dans Proc. Natl. Acad. Sci. USA, 1983, 80, 31-35.

On obtient une banque d'ADNc contenant environ 30 000 clones recombinants.

### Vecteur.

On utilise des plasmides pEX1-3 (voir Stanley et Luzio, EMBO Journal, 1984, 3, 1429-1430, 1984). Ces vecteurs dérivent de plasmides contenant le gène de fusion cro-LacZ, dont l'expression est sous contrôle du promoteur PR du bactériophage lambda. Un polynucléotide contenant plusieurs sites uniques de restriction a été inséré à l'extrémité 3' du gène LacZ. Les signaux de terminaison de transcription et de traduction sont également insérés dans les trois phases de lecture.

Les ADNc ont été insérés dans chacun des plasmides pEX digérés par les enzymes de restriction Bam H1 SalI. Ils se trouvent donc tous dans la même orientation par rapport au gène CroLacZ.

### Transformation.

On utilise comme souche bactérienne la souche E. coli pop 2135, construite par O. Raibaud comme décrit dans Nucleic Acid Research de la manière suivante :

On clone dans le site Bam HI d'un polylinker le fragment BglII de 2,3 kb du phage lambda, portant l'allèle C1857 et le promoteur P_{R} en opérant selon le schéma ci-dessous :

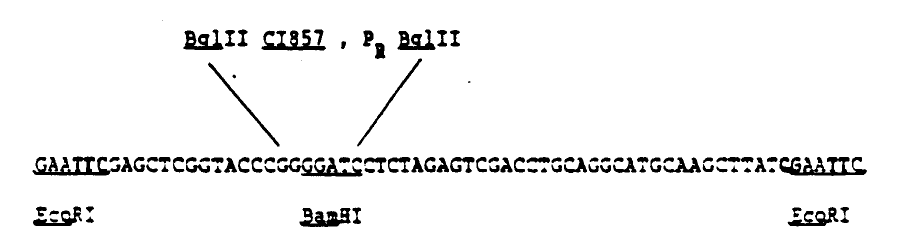
BglII CI857 , PR BglII


GAATTCGAGCTCGGTACCCGGGGATCCTCTAGAGTCGACCTGCAGGCATGCAAGCTTATCGAATTC

EcoRI BamHI EcoRI

Le fragment EcoRI ainsi obtenu est alors cloné dans le site EcoRI de pOM41 et transféré dans le chromosome de la souche C600 (voir Gene, 29, 231-241).

Par cotransduction P1' avec un marqueur proximal aroB, on introduit cette structure dans le chromosone de MM294 (F⁻ endA thi hsdR). On obtient E. coli pop 2135 : orientation maIT, CI857, P_{R}, maIPO.

La transformation de la souche bactérienne E. coli pop 2135 par les plasmides recombinants est réalisée selon la technique utilisant le rubidium décrite par Hanahan et al., J. Mol. Biol., 1983, 166, 557-580. Cette souche contient l'allèle cIts857 du répresseur de cro.

Le nombre de recombinants obtenus est de l'ordre de 10³ à 10⁴ ng d'ADNc.

### Sélection des clones par détection immunologique.

Les clones recombinants obtenus sont étalés sur filtres de nitrocellulose. La synthèse de la protéine hybride est induite après 2 heures d'incubation à 42°C. Après lyse des bactéries par le SDS et renaturation des protéines en l'absence de méthanol, les clones sont analysés par criblage immunologique. La renaturation des protéines est effectuée selon la technique de Burnett rapportée dans Anal. Biochem. 1981, 112, 195-203.

Dans un premier temps, la banque est criblée par un anticorps polyclonal dirigé contre la villine de porc intacte. On effectue ensuite un criblage secondaire en utilisant un anticorps polyclonal dirigé contre un fragment COOH-terminal de la villine de poulet et deux anticorps monoclonaux (BDD₂C₃ et IID₃H₉) reconnaissant les épitopes situés dans la région COOH-terminale de la molécule.

Le clone pEXZ-V19 contient un insérat de 510 paires de bases. La partie codante représente 330 paires de bases. Elle est suivie d'une région non codante de 200 paires de bases.

L'ADNc cloné code pour les 95 acides aminés COOH-terminaux de la villine humaine et représente environ le 1/10° de la protéine entière (poids moléculaire : 95 Kd).

### REFERENCES

(1) Ullrich, A., Shine, J., Chirgwin, J., Pictet, R., Tischer, E., Rutter, W.J. & Goodman, H.M.
   Rat insulin genes : construction of plasmids containing the coding sequences.
   Science, 1977, 196, 1313-1319.
(2) Aviv, H. & Leder, P.
   Purification of biologically active globin mRNA by chromatography on oligothymidylic acid cellulose.
   Proc. Natl. Acad. Sci. USA, 1972, 69, 1408-1412.
(3) Fiddes, J.C. & Goodman, H.M.
   Isolation, cloning and sequence analysis of the cDNA for the alpha-subunit of human chorionic gonadotropin.
   Nature, 1979, 281, 351-355.
(4) Helfman, D.M., Feramisco, J.R., Fiddes, J.C., Thomas, G.P. & Hughes, S.H.
   Identification of clones that encode chicken tropomyosin by direct immunological screening of a cDNA expression library.
   Proc. Natl. Acad. Sci. USA, 1983, 80, 31-35.
(5) Stanley, K.K. & Luzio, J.P.
   Construction of a new family of high efficiency bacterial expression vectors : identification of cDNA clones coding for human liver.
   EMBO Journal, 1984, 3, 1429-1434.
(6) Stanley K.K.
   Solubilization and immune-detection of beta-galactosidase hybrid proteins carrying foreign antigenic determinants.
   Nucleic Acids Res., 1983, 11, 4077-4092.
(7) Hanahan, D.
   Studies on transformation of E. coli with plasmids. J. Mol. Biol., 1983, 166, 557-580.
(8) Burnett, W.N.
   "Western Blotting" : Electrophoretic transfer of proteins from sodium dodecyl sulfate-polyacrylamide gels to unmodified nitrocellulose and radiographic detection with antibody and radioiodinated protein A.
   Anal. Biochem., 1981, 112, 195-203.

## Revendications (Revendications pour l'(les) Etat(s) contractant(s) suivant(s): BE, CH, LI, DE, FR, GB, IT, LU, NL, SE)

1. Villine humaine biologiquement pure, caractérisée en ce qu'elle ne donne essentiellement qu'une seule bande de poids moléculaire de 95 kD dans les essais de migration en électrophorèse sur gel de polyacrylamide et caractérisée en ce que son extrémité COOH-terminale comporte la séquence d'acides aminés suivante :

LYS TRP SER ASN THR LYS SER TYR GLU ASP LEU LYS ALA GLU
SER GLY ASN SER ARG ASP TRP SER GLN ILE THR ALA GLU VAL
THR SER PRO LYS VAL ASP VAL PHE ASN ALA ASN SER ASN LEU
SER SER GLY PRO LEU PRO ILE PHE PRO LEU GLU GLN LEU VAL
ASN LYS PRO VAL GLU GLU LEU PRO GLU GLY VAL ASP PRO SER
ARG LYS GLU GLU HIS LEU SER ILE GLU ASP PHE THR GLN ALA
PHE GLY MET THR PRO ALA ALA PHE SER ALA LEU PRO ARG TRP
LYS GLN GLN ASN LEU LYS LYS GLU LYS GLY LEU PHE

2. Fragment polypeptidique de la villine humaine, caractérisé en ce qu'il est reconnu par des anticorps reconnaissant la villine humaine biologiquement pure ne donnant essentiellement qu'une seule bande de poids moléculaire de 95 kD, dans les essais de migration en électrophorèse sur gel de polyacrylamide.

3. Fragment de la villine humaine selon la revendication 2, caractérisé en ce qu'il est immunogène.

4. Fragment polypeptidique de la villine humaine selon la revendication 2, caractérisé en ce qu'il est codé par l'ADN correspondant à l'ARNm complet de la villine humaine ou par un fragment d'ADN, capable de s'hybrider spécifiquement avec une séquence nucléotidique codant pour la villine.

5. Fragment polypeptidique selon la revendication 4, caractérisé en ce qu'il répond à la séquence suivante ou à toute partie de cette séquence reconnue par des anticorps reconnaissant la villine humaine.

LYS TRP SER ASN THR LYS SER TYR GLU ASP LEU LYS ALA GLU
SER GLY ASN SER ARG ASP TRP SER GLN ILE THR ALA GLU VAL
THR SER PRO LYS VAL ASP VAL PHE ASN ALA ASN SER ASN LEU
SER SER GLY PRO LEU PRO ILE PHE PRO LEU GLU GLN LEU VAL
ASN LYS PRO VAL GLU GLU LEU PRO GLU GLY VAL ASP PRO SER
ARG LYS GLU GLU HIS LEU SER ILE GLU ASP PHE THR GLN ALA
PHE GLY MET THR PRO ALA ALA PHE SER ALA LEU PRO ARG TRP
LYS GLN GLN ASN LEU LYS LYS GLU LYS GLY LEU PHE

6. Anticorps monoclonal, caractérisé en ce qu'il reconnait la villine humaine biologiquement pure ne donnant essentiellement qu'une seule bande de poids moléculaire de 95 kD, dans les essais de migration en électrophorèse sur gel de polyacrylamide.

7. Anticorps monoclonal selon la revendication 6, caractérisé en ce qu'il reconnait une séquence d'acides aminés selon l'une quelconque des revendications 1 à 5.

8. Anticorps monoclonal selon la revendication 6 dirigé contre la villine, caractérisé par les propriétés suivantes :
- il reconnait la villine de porc intacte purifiée (Western Blotting),
- il reconnait (Western Blotting) l'extrémité COOH-terminale de la villine humaine selon la revendication 2 ou la revendication 3,
- il reconnait la villine de rat en immunocytochimie (coupes congelées (cryostat) de muqueuse intestinale de rat fixées à la formaldéhyde),
- il reconnait (Western Blotting) un site antigénique contenu dans la séquence C-terminale (779-854) ou "partie de tête" de la villine de poulet de formule : 
1                                                    10   11
Val-Phe-Thr-Ala-Thr-Thr-Thr-Leu-Val-Pro-Thr-Lys-Leu-Glu
                        20
Thr-Phe-Pro-Leu-Asp-Val-Leu-Val-Asn-Thr-Ala-Ala-Glu-Asp
    30   31                                        40   41
Leu-Pro-Arg-Gly-Val-Asp-Pro-Ser-Arg-Lys-Glu-Asn-His-Leu
                                    50   51
Ser-Asp-Glu-Asp-Phe-Lys-Ala-Val-Phe-Gly-Met-Thr-Arg-Ser
    60   61                                        70   71
Ala-Asn-Leu-Pro-Leu-Trp-Lys-Gln-Gln-Asn-Leu-Lys-Lys-Glu

Glu-Lys-Gly-Leu-Phe
ledit anticorps monoclonal étant capable de reconnaître ladite "partie de tête", que celle-ci soit isolée ou à l'intérieur du peptide 51 kD contenu dans la villine du poulet, que cette "partie de tête", soit dénaturée, ou présente sur des cellules épithéliales porteuses de la villine.

## Revendications (Revendications pour l'(les) Etat(s) contractant(s) suivant(s): AT)

1. Procédé de préparation de la villine humaine biologiquement pure caractérisé en ce qu'il comprend
- le traitement d'un lysat d'entérocytes humains avec une solution acqueuse contenant un détergent approprié et la récupération d'une solution contenant la villine humaine,
- le passage de la solution contenant la villine humaine au contact d'une colonne d'affinité portant un anticorps monoclonal dirigé contre la villine humaine tel qu'obtenu à partir de l'hybridome CNCM I-440, dans des conditions permettant la fixation sélective de la villine humaine par l'anticorps,
- la récupération de la villine humaine par dissociation du complexe antigène-anticorps, ladite villine ne donnant essentiellement qu'une seule bande de poids moléculaire de 95 kD dans les essais de migration en électrophorèse sur gel de polyacrylamide et comportant à son extrémité COOH-terminale, la séquence d'acides aminés suivante :

LYS TRP SER ASN THR LYS SER TYR GLU ASP LEU LYS ALA GLU
SER GLY ASN SER ARG ASP TRP SER GLN ILE THR ALA GLU VAL
THR SER PRO LYS VAL ASP VAL PHE ASN ALA ASN SER ASN LEU
SER SER GLY PRO LEU PRO ILE PHE PRO LEU GLU GLN LEU VAL
ASN LYS PRO VAL GLU GLU LEU PRO GLU GLY VAL ASP PRO SER
ARG LYS GLU GLU HIS LEU SER ILE GLU ASP PHE THR GLN ALA
PHE GLY MET THR PRO ALA ALA PHE SER ALA LEU PRO ARG TRP
LYS GLN GLN ASN LEU LYS LYS GLU LYS GLY LEU PHE

2. Procédé de préparation d'un fragment polypeptidique de la villine humaine caractérisé en ce qu'il comprend
- le traitement d'un lysat d'entérocytes humains avec une solution acqueuse contenant un détergent approprié et la récupération d'une solution contenant la villine humaine,
- le passage de la solution contenant la villine humaine au contact d'une colonne d'affinité portant un anticorps monoclonal dirigé contre la villine humaine tel qu'obtenu à partir de l'hybridome CNCM I-440, dans des conditions permettant la fixation sélective de la villine humaine par l'anticorps,
- la récupération de la villine humaine par dissociation du complexe antigène-anticorps,
- la coupure enzymatique de la villine humaine obtenue, pour produire un fragment reconnu par des anticorps reconnaissant la villine humaine biologiquement pure ne donnant essentiellement qu'une seule bande de poids moléculaire de 95 kD, dans les essais de migration en électrophorèse sur gel de polyacrylamide.

3. Procédé de préparation d'un fragment de la villine humaine selon la revendication 2, caractérisé en ce que le fragment obtenu est immunogène.

4. Procédé de préparation d'un fragment polypeptidique de la villine humaine selon la revendication 2, caractérisé en ce que le fragment obtenu est codé par l'ADN correspondant à l'ARNm complet de la villine humaine ou par un fragment d'ADN, capable de s'hybrider spécifiquement avec une séquence nucléotidique codant pour la villine.

5. Procédé de préparation d'un fragment polypeptidique selon la revendication 4, caractérisé en ce que le fragment obtenu répond à la séquence suivante ou à toute partie de cette séquence reconnue par des anticorps reconnaissant la villine humaine.

LYS TRP SER ASN THR LYS SER TYR GLU ASP LEU LYS ALA GLU
SER GLY ASN SER ARG ASP TRP SER GLN ILE THR ALA GLU VAL
THR SER PRO LYS VAL ASP VAL PHE ASN ALA ASN SER ASN LEU
SER SER GLY PRO LEU PRO ILE PHE PRO LEU GLU GLN LEU VAL
ASN LYS PRO VAL GLU GLU LEU PRO GLU GLY VAL ASP PRO SER
ARG LYS GLU GLU HIS LEU SER ILE GLU ASP PHE THR GLN ALA
PHE GLY MET THR PRO ALA ALA PHE SER ALA LEU PRO ARG TRP
LYS GLN GLN ASN LEU LYS LYS GLU LYS GLY LEU PHE

6. Procédé de préparation d'un anticorps monoclonal, comprenant :
- la fusion de cellules spléniques d'une souris préalablement immunisée avec la villine de porc, avec des cellules de myélome,
- la sélection des hybridomes sécréteurs d'un anticorps monoclonal, l'anticorps produit reconnaissant la villine humaine biologiquement pure et ne donnant essentiellement qu'une seule bande de poids moléculaire de 95 kD, dans les essais de migration en électrophorèse sur gel de polyacrylamide.

7. Procédé de préparation d'un anticorps monoclonal selon la revendication 6, caractérisé en ce que l'anticorps obtenu reconnaît une séquence d'acides aminés selon l'une quelconque des revendications 1 à 5.

8. Procédé de préparation d'un anticorps monoclonal selon la revendication 6 dirigé contre la villine, caractérisé en ce que l'anticorps obtenu a les propriétés suivantes :
- il reconnaît la villine de porc intacte purifiée (Western Blotting),
- il reconnait (Western Blotting) l'extrémité COOH-terminale de la villine humaine selon la revendication 2 ou la revendication 3,
- il reconnaît la villine de rat en immunocytochimie (coupes congelées (cryostat) de muqueuse intestinale de rat fixées à la formaldéhyde),
- il reconnaît (Western Blotting) un site antigénique contenu dans la séquence C-terminale (779-854) ou "partie de tête" de la villine de poulet de formule : 
1                                                    10   11
Val-Phe-Thr-Ala-Thr-Thr-Thr-Leu-Val-Pro-Thr-Lys-Leu-Glu
                        20
Thr-Phe-Pro-Leu-Asp-Val-Leu-Val-Asn-Thr-Ala-Ala-Glu-Asp
    30   31                                      40   41
Leu-Pro-Arg-Gly-Val-Asp-Pro-Ser-Arg-Lys-Glu-Asn-His-Leu
                                    50   51
Ser-Asp-Glu-Asp-Phe-Lys-Ala-Val-Phe-Gly-Met-Thr-Arg-Ser
    60   61                                      70   71
Ala-Asn-Leu-Pro-Leu-Trp-Lys-Gln-Gln-Asn-Leu-Lys-Lys-Glu

Glu-Lys-Gly-Leu-Phe
ledit anticorps monoclonal étant capable de reconnaître ladite "partie de tête", que celle-ci soit isolée ou à l'intérieur du peptide 51 kD contenu dans la villine du poulet, que cette "partie de tête", soit dénaturée, ou présente sur des cellules épithéliales porteuses de la villine.

## Patentansprüche (Patentansprüche für folgende(n) Vertragsstaat(en): BE, CH, LI, DE, FR, GB, IT, LU, NL, SE)

1. Biologisch reines menschliches Villin, dadurch gekennzeichnet, daß es bei der Untersuchung der Wanderung bei der Elektrophorese auf Polyacrylamidgel im wesentlichen nur eine einzige Bande mit einem Molekulargewicht von 95 kD ergibt und dadurch gekennzeichnet ist, daß sein COOH-terminales Ende die folgende aminosäuresequenz trägt:

LYS TRP SER ASN THR LYS SER TYR GLU ASP LEU LYS ALA GLU
SER GLY ASN SER ARG ASP TRP SER GLN ILE THR ALA GLU VAL
THR SER PRO LYS VAL ASP VAL PHE ASN ALA ASN SER ASN LEU
SER SER GLY PRO LEU PRO ILE PHE PRO LEU GLU GLN LEU VAL
ASN LYS PRO VAL GLU GLU LEU PRO GLU GLY VAL ASP PRO SER
ARG LYS GLU GLU HIS LEU SER ILE GLU ASP PHE THR GLN ALA
PHE GLY MET THR PRO ALA ALA PHE SER ALA LEU PRO ARG TRP
LYS GLN GLN ASN LEU LYS LYS GLU LYS GLY LEU PHE

2. Polypeptidfragment des menschlichen Villins, dadurch gekennzeichnet, daß es von Antikörpern erkannt wird, welche biologisch reines menschliches Villin erkennen, das bei der Untersuchung der Wanderung bei der Elektrophorese auf Polyacrylamidgel im wesentlichen nur eine einzige Bande mit einem Molekulargewicht von 95 kD ergibt.

3. Menschliches Villinfragment nach Anspruch 2, dadurch gekennzeichnet, daß es immunogen ist.

4. Polypeptidfragment des menschlichen Villins nach Anspruch 2, dadurch gekennzeichnet, daß es durch die DNA codiert wird, welche der vollständigen mRNA des menschlichen Villins entspricht, oder durch ein DNA-Fragment, welches dazu in der Lage ist, spezifisch mit einer Villin-codierenden Nucleotidsequenz zu hybridisieren.

5. Polypeptidfragment nach Anspruch 4, dadurch gekennzeichnet, daß es der folgenden Sequenz entspricht oder jeglichem Teil diese Sequenz, welcher von Antikörpern erkannt wird, die menschliches Villin erkennen:

LYS TRP SER ASN THR LYS SER TYR GLU ASP LEU LYS ALA GLU
SER GLY ASN SER ARG ASP TRP SER GLN ILE THR ALA GLU VAL
THR SER PRO LYS VAL ASP VAL PHE ASN ALA ASN SER ASN LEU
SER SER GLY PRO LEU PRO ILE PHE PRO LEU GLU GLN LEU VAL
ASN LYS PRO VAL GLU GLU LEU PRO GLU GLY VAL ASP PRO SER
ARG LYS GLU GLU HIS LEU SER ILE GLU ASP PHE THR GLN ALA
PHE GLY MET THR PRO ALA ALA PHE SER ALA LEU PRO ARG TRP
LYS GLN GLN ASN LEU LYS LYS GLU LYS GLY LEU PHE

6. Monoklonaler Antikörper, dadurch gekennzeichnet, daß er biologisch reines menschliches Villin erkennt, welches bei der Untersuchung der Wanderung bei der Elektrophorese auf Polyacrylamidgel im wesentlichen nur eine einzige Bande mit einem Molekulargewicht von 95 kD ergibt.

7. Monoklonaler Antikörper nach Anspruch 6, dadurch gekennzeichnet, daß er eine Aminosäuresequenz nach einem der Ansprüche 1 bis 5 erkennt.

8. Gegen Villin gerichteter monoklonaler Antikörper nach Anspruch 6, gekennzeichnet durch die folgenden Eigenschaften:
- er erkennt gereinigtes intaktes Schweine-Villin (Western Blotting),
- er erkennt (Western Blotting) das COOH-terminale Ende des menschlichen Villins nach Anspruch 2 oder nach Anspruch 3,
- er erkennt Ratten-Villin bei der Immunocytochemie (gefrorene Schnitte (Cryostat) der Darmschleimhaut von Ratten, welche mit Formaldehyd fixiert worden ist),
- er erkennt (Western Blotting) eine antigene Stelle, welche in der C-terminalen Sequenz (779-854) oder dem "Kopfteil" des Hühner-Villins der Formel: 
1                                   10  11
Val-Phe-Thr-Ala-Thr-Thr-Thr-Leu-Val-Pro-Thr-Lys-Leu-Glu
                    20
Thr-Phe-Pro-Leu-Asp-Val-Leu-Val-Asn-Thr-Ala-Ala-Glu-Asp
    30  31                                  40  41
Leu-Pro-Arg-Gly-Val-Asp-Pro-Ser-Arg-Lys-Glu-Asn-His-Leu
                                50  51
Ser-Asp-Glu-Asp-Phe-Lys-Ala-Val-Phe-Gly-Met-Thr-Arg-Ser
    60  61                                  70  71
Ala-Asn-Leu-Pro-Leu-Trp-Lys-Gln-Gln-Asn-Leu-Lys-Lys-Glu

Glu-Lys-Gly-Leu-Phe
 enthalten ist,
welcher monoklonale Antikörper dazu in der Lage ist, den genannten "Kopfteil" zu erkennen, wenn dieser isoliert ist oder im Inneren des 51 kD-Peptids vorliegt, welches im Hühner-Villin enthalten ist, welcher "Kopfteil" entweder denaturiert ist oder auf Epithelialzellen vorliegt, die das Villin tragen.

## Patentansprüche (Patentansprüche für folgende(n) Vertragsstaat(en): AT)

1. Verfahren zur Herstellung von biologisch reinem menschlichem Villin, dadurch gekennzeichnet, daß es umfaßt:
- die Behandlung eines Lysats von menschlichen Enterocyten mit einer wäßrigen Lösung, welche ein geeignetes Detergens enthält, und Gewinnung einer das menschliche Villin enthaltenden Lösung,
- das Hindurchführen der das menschliche Villin enthaltenden Lösung durch und im Kontakt mit einer Affinitätssäule, welche einen monoklonalen Antikörper trägt, der gegen das menschliche Villin gerichtet ist, wie er ausgehend von dem Hybridom CNCMI-440 erhalten worden ist, unter Bedingungen, welche die selektive Bindung des menschlichen Villins durch den Antikörper ermöglichen,
- die Gewinnung des menschlichen Villins durch Dissoziation des Antigen-Antikörper-Komplexes, wobei das Villin bei der Untersuchung der Wanderung bei der Elektrophorese auf Polyacrylamidgel im wesentlichen nur eine einzige Bande mit einem Molekulargewicht von 95 kD ergibt, und welches an seinem COOH-terminalen Ende die folgende Aminosäuresequenz aufweist:

LYS TRP SER ASN THR LYS SER TYR GLU ASP LEU LYS ALA GLU
SER GLY ASN SER ARG ASP TRP SER GLN ILE THR ALA GLU VAL
THR SER PRO LYS VAL ASP VAL PHE ASN ALA ASN SER ASN LEU
SER SER GLY PRO LEU PRO ILE PHE PRO LEU GLU GLN LEU VAL
ASN LYS PRO VAL GLU GLU LEU PRO GLU GLY VAL ASP PRO SER
ARG LYS GLU GLU HIS LEU SER ILE GLU ASP PHE THR GLN ALA
PHE GLY MET THR PRO ALA ALA PHE SER ALA LEU PRO ARG TRP
LYS GLN GLN ASN LEU LYS LYS GLU LYS GLY LEU PHE

2. Verfahren zur Herstellung eines Polypeptidfragments von menschlichem Villin, dadurch gekennzeichnet, daß es umfaßt:
- die Behandlung eines Lysats von menschlichen Enterocyten mit einer wäßrigen Lösung, die ein geeignetes Detergens enthält, und Gewinnung einer das menschliche Villin enthaltenden Lösung,
- das Hindurchführen der das menschliche Villin enthaltenden Lösung durch und im Kontakt mit einer Affinitätssäule, welche einen monoklonalen Antikörper trägt, der gegen das menschliche Villin gerichtet ist, wie er ausgehend von dem Hybridom CNCMI-440 erhalten worden ist, unter Bedingungen, welche die selektive Bindung des menschlichen Villins durch den Antikörper ermöglichen,
- die Gewinnung des menschlichen Villins durch Dissoziation des Antigen-Antikörper-Komplexes,
- die enzymatische Spaltung des erhaltenen menschlichen Villins zur Bildung eines Fragments, welches von Antikörpern erkannt wird, die das biologisch reine Villin erkennen, das bei der Untersuchung der Wanderung bei der Elektrophorese auf Polyacrylamidgel im wesentlichen nur eine einzige Bande mit einem Molekulargewicht von 95 kD ergibt.

3. Verfahren zur Herstellung eines menschlichen Villinfragments nach Anspruch 2, dadurch gekennzeichnet, daß das erhaltene Fragment immunogen ist.

4. Verfahren zur Herstellung eines Polypeptidfragments des menschlichen Villins nach Anspruch 2, dadurch gekennzeichnet, daß das erhaltene Fragment durch die DNA codiert wird, welche der vollständigen mRNA des menschlichen Villins entspricht, oder durch ein DNA-Fragment, welches dazu in der Lage ist, spezifisch mit einer das Villin codierenden Nucleotidsequenz zu hybridisieren.

5. Verfahren zur Herstellung eines Polypeptidsfragments nach Anspruch 4, dadurch gekennzeichnet, daß das erhaltene Fragment der folgenden Sequenz entspricht oder jedem Teil dieser Sequenz, der durch Antikörper erkannt wird, welche menschliches Villin erkennen:

LYS TRP SER ASN THR LYS SER TYR GLU ASP LEU LYS ALA GLU
SER GLY ASN SER ARG ASP TRP SER GLN ILE THR ALA GLU VAL
THR SER PRO LYS VAL ASP VAL PHE ASN ALA ASN SER ASN LEU
SER SER GLY PRO LEU PRO ILE PHE PRO LEU GLU GLN LEU VAL
ASN LYS PRO VAL GLU GLU LEU PRO GLU GLY VAL ASP PRO SER
ARG LYS GLU GLU HIS LEU SER ILE GLU ASP PHE THR GLN ALA
PHE GLY MET THR PRO ALA ALA PHE SER ALA LEU PRO ARG TRP
LYS GLN GLN ASN LEU LYS LYS GLU LYS GLY LEU PHE

6. Verfahren zur Herstellung eines monoklonalen Antikörpers, umfassend:
- die Fusion von Milzzellen einer Maus, die zuvor mit Schweine-Villin immunisiert worden ist, mit Myelomzellen,
- die Selektion von Hybridomen, welche einen monoklonalen Antikörper sekretieren, welcher gebildete Antikörper biologisch reines menschliches Villin erkennt, das bei der Untersuchung der Wanderung bei der Elektrophorese auf Polyacrylamidgel im wesentlichen nur eine einzige Bande mit einem Molekulargewicht von 95 kD ergibt.

7. Verfahren zur Herstellung eines monoklonalen Antikörpers nach Anspruch 6, dadurch gekennzeichnet, daß der erhaltene Antikörper eine Aminosäuresequenz nach einem der Ansprüche 1 bis 5 erkennt.

8. Verfahren zur Herstellung eines monoklonalen Antikörpers nach Anspruch 6, der gegen Villin gerichtet ist, dadurch gekennzeichnet, daß der erhaltene Antikörper die folgenden Eigenschaften besitzt:
- er erkennt gereinigtes intaktes Schweine-Villin (Western Blotting),
- er erkennt (Western Blotting) das COOH-terminale Ende des menschlichen Villins nach Anspruch 2 oder nach Anspruch 3,
- er erkennt Ratten-Villin bei der Immunocytochemie (gefrorene Schnitte (Cryostat) der Darmschleimhaut von Ratten, welche mit Formaldehyd fixiert worden ist),
- er erkennt (Western Blotting) eine antigene Stelle, welche in der C-terminalen Sequenz (779-854) oder dem "Kopfteil" des Hühner-Villins der Formel: 
1                                       10  11
Val-Phe-Thr-Ala-Thr-Thr-Thr-Leu-Val-Pro-Thr-Lys-Leu-Glu
                    20
Thr-Phe-Pro-Leu-Asp-Val-Leu-Val-Asn-Thr-Ala-Ala-Glu-Asp
    30  31                                  40  41
Leu-Pro-Arg-Gly-Val-Asp-Pro-Ser-Arg-Lys-Glu-Asn-His-Leu
                                50  51
Ser-Asp-Glu-Asp-Phe-Lys-Ala-Val-Phe-Gly-Met-Thr-Arg-Ser
    60  61                                  70  71
Ala-Asn-Leu-Pro-Leu-Trp-Lys-Gln-Gln-Asn-Leu-Lys-Lys-Glu

Glu-Lys-Gly-Leu-Phe
 enthalten ist,
welcher monoklonale Antikörper dazu in der Lage ist, den genannten "Kopfteil" zu erkennen, wenn dieser isoliert ist oder im Inneren des 51 kD-Peptids vorliegt, welches im Hühner-Villin enthalten ist, welcher "Kopfteil" entweder denaturiert ist oder auf Epithelialzellen vorliegt, die das Villin tragen.

## Claims (Claims for the following Contracting State(s): BE, CH, LI, DE, FR, GB, IT, LU, NL, SE)

1. Biologically pure human villin, characterized in that it essentially gives only one band of 95 kD molecular weight in electrophoretic migration tests on polyacrylamide gel, and characterized in that its COOH-terminal end comprises the following amino acid sequence:

LYS TRP SER ASN THR LYS SER TYR GLU ASP LEU LYS ALA GLU
SER GLY ASN SER ARG ASP TRP SER GLN ILE THR ALA GLU VAL
THR SER PRO LYS VAL ASP VAL PHE ASN ALA ASN SER ASN LEU
SER SER GLY PRO LEU PRO ILE PHE PRO LEU GLU GLN LEU VAL
ASN LYS PRO VAL GLU GLU LEU PRO GLU GLY VAL ASP PRO SER
ARG LYS GLU GLU HIS LEU SER ILE GLU ASP PHE THR GLN ALA
PHE GLY MET THR PRO ALA ALA PHE SER ALA LEU PRO ARG TRP
LYS GLN GLN ASN LEU LYS LYS GLU LYS GLY LEU PHE

2. Polypeptide fragment of human villin, characterized in that it is recognized by antibodies which recognize biologically pure human villin which essentially gives only one band of 95 kD molecular weight in electrophoretic migration tests on polyacrylamide gel.

3. Fragment of human villin according to Claim 2, characterized in that it is immunogenic.

4. Polypeptide fragment of human villin according to Claim 2, characterized in that it is encoded by the DNA which corresponds to the complete mRNA of human villin, or by a DNA fragment which is able to hybridize specifically with a nucleotide sequence encoding villin.

5. Polypeptide fragment according to Claim 4, characterized in that it corresponds to the following sequence or any part of this sequence which is recognized by antibodies which recognize human villin.

LYS TRP SER ASN THR LYS SER TYR GLU ASP LEU LYS ALA GLU
SER GLY ASN SER ARG ASP TRP SER GLN ILE THR ALA GLU VAL
THR SER PRO LYS VAL ASP VAL PHE ASN ALA ASN SER ASN LEU
SER SER GLY PRO LEU PRO ILE PHE PRO LEU GLU GLN LEU VAL
ASN LYS PRO VAL GLU GLU LEU PRO GLU GLY VAL ASP PRO SER
ARG LYS GLU GLU HIS LEU SER ILE GLU ASP PHE THR GLN ALA
PHE GLY MET THR PRO ALA ALA PHE SER ALA LEU PRO ARG TRP
LYS GLN GLN ASN LEU LYS LYS GLU LYS GLY LEU PHE

6. Monoclonal antibody, characterized in that it recognizes biologically pure human villin which essentially gives only one band of 95 kD molecular weight in electrophoretic migration tests on polyacrylamide gel.

7. Monoclonal antibody according to Claim 6, characterized in that it recognizes an amino acid sequence according to any one of Claims 1 to 5.

8. Monoclonal antibody according to Claim 6 which is directed against villin, characterized by the following properties:
- it recognizes purified intact porcine villin (Western blotting),
- it recognizes (Western blotting) the COOH-terminal end of human villin according to Claim 2 or Claim 3,
- it recognizes rat villin in immunocytochemistry (frozen sections (cryostat) of rat intestinal mucosa fixed with formaldehyde),
- it recognizes (Western blotting) an antigenic site which is contained in the C-terminal sequence (779-854) or "head part" of chick villin of the formula: 
1                                       10  11
Val-Phe-Thr-Ala-Thr-Thr-Thr-Leu-Val-Pro-Thr-Lys-Leu-Glu
                    20
Thr-Phe-Pro-Leu-Asp-Val-Leu-Val-Asn-Thr-Ala-Ala-Glu-Asp
    30  31                                  40  41
Leu-Pro-Arg-Gly-Val-Asp-Pro-Ser-Arg-Lys-Glu-Asn-His-Leu
                            50  51
Ser-Asp-Glu-Asp-Phe-Lys-Ala-Val-Phe-Gly-Met-Thr-Arg-Ser
    60  61                                  70  71
Ala-Asn-Leu-Pro-Leu-Trp-Lys-Gln-Gln-Asn-Leu-Lys-Lys-Glu

Glu-Lys-Gly-Leu-Phe
the said monoclonal antibody being able to recognize the said "head part", whether the latter is isolated or in the interior of the 51 kD peptide which is contained in chick villin, and whether this "head part" is denatured or present on epithelial cells which are carrying the villin.

## Claims (Claims for the following Contracting State(s): AT)

1. Process for preparing biologically pure human villin, characterized in that it comprises
- treating a lysate of human enterocytes with an aqueous solution containing a suitable detergent and recovering a solution containing human villin,
- bringing the solution containing the human villin into contact with an affinity column carrying a monoclonal antibody directed against human villin, such as obtained from the hybridoma CNCM I-440, under conditions which permit selective binding of the human villin by the antibody,
- recovering the human villin by dissociation of the antigen/antibody complex, with the said villin essentially giving only one band of 95 kD molecular weight in electrophoretic migration tests on polyacrylamide gel and comprising, at its COOH-terminal end, the following amino acid sequence:

LYS TRP SER ASN THR LYS SER TYR GLU ASP LEU LYS ALA GLU
SER GLY ASN SER ARG ASP TRP SER GLN ILE THR ALA GLU VAL
THR SER PRO LYS VAL ASP VAL PHE ASN ALA ASN SER ASN LEU
SER SER GLY PRO LEU PRO ILE PHE PRO LEU GLU GLN LEU VAL
ASN LYS PRO VAL GLU GLU LEU PRO GLU GLY VAL ASP PRO SER
ARG LYS GLU GLU HIS LEU SER ILE GLU ASP PHE THR GLN ALA
PHE GLY MET THR PRO ALA ALA PHE SER ALA LEU PRO ARG TRP
LYS GLN GLN ASN LEU LYS LYS GLU LYS GLY LEU PHE

2. Process for preparing a polypeptide fragment of human villin, characterized in that it comprises
- treating a lysate of human enterocytes with an aqueous solution containing a suitable detergent and recovering a solution containing human villin,
- bringing the solution containing the human villin into contact with an affinity column carrying a monoclonal antibody directed against human villin, such as obtained from the hybridoma CNCM I-440, under conditions which permit selective binding of the human villin by the antibody,
- recovering the human villin by dissociation of the antigen/antibody complex,
- enzymatically cleaving the human villin which has been obtained in order to produce a fragment which is recognized by antibodies which recognize biologically pure human villin which essentially gives only one band of 95 kD molecular weight in electrophoretic migration tests on polyacrylamide gel.

3. Process for preparing a fragment of human villin according to Claim 2, characterized in that the fragment which is obtained is immunogenic.

4. Process for preparing a polypeptide fragment of human villin according to Claim 2, characterized in that the fragment which is obtained is encoded by the DNA which corresponds to the complete mRNA of human villin or by a DNA fragment which is able to hybridize specifically with a nucleotide sequence encoding villin.

5. Process for preparing a polypeptide fragment according to Claim 4, characterized in that the fragment which is obtained corresponds to the following sequence or any part of this sequence which is recognized by antibodies which recognize human villin.

LYS TRP SER ASN THR LYS SER TYR GLU ASP LEU LYS ALA GLU
SER GLY ASN SER ARG ASP TRP SER GLN ILE THR ALA GLU VAL
THR SER PRO LYS VAL ASP VAL PHE ASN ALA ASN SER ASN LEU
SER SER GLY PRO LEU PRO ILE PHE PRO LEU GLU GLN LEU VAL
ASN LYS PRO VAL GLU GLU LEU PRO GLU GLY VAL ASP PRO SER
ARG LYS GLU GLU HIS LEU SER ILE GLU ASP PHE THR GLN ALA
PHE GLY MET THR PRO ALA ALA PHE SER ALA LEU PRO ARG TRP
LYS GLN GLN ASN LEU LYS LYS GLU LYS GLY LEU PHE

6. Process for preparing a monoclonal antibody, comprising:
- fusing spleen cells from a mouse which has previously been immunized with porcine villin with myeloma cells,
- selecting hybridomas which secrete a monoclonal antibody, with the antibody which is produced recognizing human villin which is biologically pure and which essentially gives only one band of 95 kD molecular weight in electrophoretic migration tests on polyacrylamide gel.

7. Process for preparing a monoclonal antibody according to Claim 6, characterized in that the antibody which is obtained recognizes an amino acid sequence according to any one of Claims 1 to 5.

8. Process for preparing a monoclonal antibody according to Claim 6 which is directed against villin, characterized in that the antibody which is obtained has the following properties:
- it recognizes purified intact porcine villin (Western blotting),
- it recognizes (Western blotting) the COOH-terminal end of human villin according to Claim 2 or Claim 3,
- it recognizes rat villin in immunocytochemistry (frozen sections (cryostat) of rat intestinal mucosa fixed with formaldehyde),
- it recognizes (Western blotting) an antigenic site which is contained in the C-terminal sequence (779-854) or "head part" of chick villin of the formula: 
1                                       10  11
Val-Phe-Thr-Ala-Thr-Thr-Thr-Leu-Val-Pro-Thr-Lys-Leu-Glu
                    20
Thr-Phe-Pro-Leu-Asp-Val-Leu-Val-Asn-Thr-Ala-Ala-Glu-Asp
    30  31                                  40  41
Leu-Pro-Arg-Gly-Val-Asp-Pro-Ser-Arg-Lys-Glu-Asn-His-Leu
                                50  51
Ser-Asp-Glu-Asp-Phe-Lys-Ala-Val-Phe-Gly-Met-Thr-Arg-Ser
    60  61                                  70  71
Ala-Asn-Leu-Pro-Leu-Trp-Lys-Gln-Gln-Asn-Leu-Lys-Lys-Glu
 Glu-Lys-Gly-Leu-Phe
the said monoclonal antibody being able to recognize the said "head part", whether the latter is isolated or in the interior of the 51 kD peptide which is contained in chick villin, and whether this "head part" is denatured or present on epithelial cells which are carrying the villin.
